# EUROPÄISCHE PATENTSCHRIFT

(11) **EP 1 894 529 B1**
(45) Veröffentlichungstag und Bekanntmachung des Hinweises auf die Patenterteilung: **21.08.2013**
(21) Anmeldenummer: 07016790.3
(22) Anmeldetag: 28.08.2007
(51) Int. Cl.: A61B 17/04

(54) **Vorrichtung zum Einführen zumindest eines Ankerstücks in einen Hohlraum eines Lebewesens**
Device for inserting at least one anchor piece into a hollow cavity of an animal
Dispositif destiné à l'introduction d'au moins un élément d'ancrage dans un espace creux d'un être vivant

(30) Priorität: 31.08.2006 DE 102006042633
(43) Veröffentlichungstag der Anmeldung: 05.03.2008
(73) Patentinhaber: Karl Storz GmbH & Co. KG, 78532 Tuttlingen (DE)
(72) Erfinder: Kohl, Thomas Dr., 53127 Bonn (DE); Oberländer, Martin, 78234 Engen (DE); Sauer, Michael, 78532 Tuttlingen (DE)
(74) Vertreter: Weller, Wolfgang

(56) Entgegenhaltungen:
- WO-A-2005/110280
- US-A- 5 085 661
- US-A- 5 626 614
- US-A- 6 110 183

## Beschreibung

Die Erfindung betrifft eine Vorrichtung zum Einführen zumindest eines Ankerstücks in einen Hohlraum eines Lebewesens, um eine Wand des Hohlraumes mittels des zumindest einen Ankerstücks zeitweilig an einer Körperwand zu fixieren, mit zwei relativ zueinander axial bewegbaren, schaftartigen Elementen, zwischen denen das zumindest eine Ankerstück unverlierbar haltbar ist, wobei eines der schaftartigen Elemente als ein Außenrohr und das andere als ein in dem Außenrohr aufgenommener, axial verschiebbarer Innenschaft ausgebildet ist, dessen distales Ende als eine Spitze ausgebildet ist, und mit einem Betätigungsmechanismus, über den zumindest ein Element derartig bewegbar ist, dass das gehaltene Ankerstück freigebbar ist.

Derartige Vorrichtungen werden im medizinischen Bereich dazu eingesetzt, die Wand eines Hohlraumes an der Körperwand während einer Untersuchung oder eines chirurgischen Eingriffs zu fixieren. Insbesondere finden sie ihren Einsatz in der fetalen Diagnostik, Therapie und Chirurgie.

Bei der fetalen Diagnostik, z.B. einer Fetoskopie, handelt es sich um eine Betrachtung eines Fötus mit Hilfe von endoskopischen Methoden.

Grundsätzlich wird dazu durch die Bauchdecke einer Schwangeren ein Beobachtungsinstrument, ein Fetoskop, in die Fruchtblase eingeführt. Mit dem Fetoskop kann ein Arzt den Fötus beobachten. Durch die Fetoskopie ist neben der Betrachtung eine Entnahme fetaler Proben (meist Haut) unter Sichtkontrolle möglich. Die entnommene fetale Probe kann mittels Spezialuntersuchungen, die zum Erkennen von Erkrankungen schon während der Schwangerschaft dienen, diagnostiziert werden.

Man verspricht sich von der fetalen Diagnostik, Therapie und Chirurgie enorme Fortschritte in der Behandlung von schweren angeborenen Fehlbildungen. Die damit verbundenen Risiken sind jedoch enorm. Die Probleme bei den oben genannten Untersuchungen und chirurgischen Eingriffen sind vielfältig. Zum einen ist es möglich, den Fötus, die Plazenta oder die Nabelschnur zu verletzen. Andererseits erhöht jede Manipulation an der Gebärmutter der Schwangeren die Gefahr für vorzeitige Wehen und eine Frühgeburt.

Um die Verletzung des Fötus, der Plazenta oder der Nabelschnur während einer fetalen Untersuchung oder eines chirurgischen Eingriffs an der Gebärmutter der Schwangeren zu vermeiden, soll in der Gebärmutter mehr Raum geschaffen werden. Zusätzlich soll die Gebärmutterwand während der Untersuchung oder des chirurgischen Eingriffs stabil und fest gehalten werden.

Durch die Ausgestaltung der schaftartigen Elemente als ein Außenrohr und als ein in dem Außenrohr aufgenommener, axial verschiebbarer Innenschaft wird eine kompakte, dennoch stabile Vorrichtung geschaffen, die auch bei insgesamt sehr geringen Baugrößen die notwendige Stabilität aufweist.

Das Ausbilden des distalen Endes des Innenschafts als eine Spitze hat den Vorteil, dass über die Spitze vom Operateur exakt der Ansatzpunkt anvisiert werden kann. Ferner ist ein sanftes Einführen der Vorrichtung durch die Bauchdecke in die Gebärmutter oder in einen Trokar möglich.

Aus der US 5,085,661 ist eine Vorrichtung zum Einführen eines Endes eines Ankerstückes in einen Hohlraum eines Lebewesens bekannt, bei der die Enden des Ankerstückes T-förmig ausgebildet sind. In einem stabförmigen Element ist eine radial relativ weit hineinreichende Aussparung vorgesehen, in die das Querstück des T-förmigen Endes des Ankerstückes eingelegt werden kann. Ein zweites hülsenartiges geschlitztes Element kann so über den vom eingelegten T-Stück vorspringenden Faden geschoben werden, dass das T-förmige Ende radial zwischen der äußeren übergeschobenen Hülse und dem inneren Stab gefangen ist. Allerdings steht der vom T-Stück vorspringende Fadenabschnitt seitlich rechtwinklig von der Vorrichtung ab.

Aus der US 5,626,614 ist eine Vorrichtung zum Einführen eines T-förmigen Ankers bekannt, bei dem distalseitig ein Rohrstück, das mit einem Faden verbunden ist, auf einen inneren Schaft aufgeschoben wird. Nach Einbringen der Vorrichtung in den Hohlraum wird das Rohrstück über einen verschiebbaren Außenschaft von dem inneren Schaft abgeschoben.

Es ist Aufgabe der vorliegenden Erfindung, eine Vorrichtung mit schlanker Bauweise zu schaffen, um ein Ankerstück sicher in den Hohlraum zu verbringen und dort freizugeben.

Erfindungsgemäß wird die Aufgabe hinsichtlich der eingangs genannten Vorrichtung dadurch gelöst, dass das zumindest eine Ankerstück an axial gegenüberliegenden Enden unverlierbar haltbar ist, dass die schaftartigen Elemente jeweils eine Backe aufweisen, zwischen denen das zumindest eine Ankerstück unverlierbar haltbar ist und dass eine Backe an dem distalen Ende des Außenrohrs und die andere Backe an dem proximalen Ende der Spitze angeordnet ist.

Diese Maßnahmen haben unter anderem den erheblichen Vorteil, dass das zumindest eine Ankerstück durch die zwei relativ zueinander axial bewegbaren, schaftartigen Elemente an zwei Stellen unverlierbar gehalten ist, und somit ein sicheres und wenig raumergreifendes Einführen des zumindest einen Ankerstücks mit Hilfe der erfindungsgemäßen Vorrichtung an eine gewünschte Stelle in der Gebärmutter der Schwangeren geschaffen wird.

Durch die Halterung des Ankerstücks an den axial gegenüberliegenden Enden müssen keine radial tief eindringenden Aussparungen vorgesehen werden, so dass beispielsweise ein Schaft als hohler Innenschaft ausgebildet werden kann, durch den ein Zieldraht durchgeschoben werden kann.

Die Ausgestaltung der schaftartigen Elemente mit Backen hat den Vorteil, dass die Backen, zwischen denen das zumindest eine Ankerstück unverlierbar gehalten ist, für einen besonders sichereren Sitz des zumindest einen Ankerstücks sorgen.

Es wird vermieden, dass das Ankerstück beim Einführen der, Vorrichtung durch die Bauchdecke der Schwangeren herausfallen kann, was zu Verletzungen und zu unnötigen Traumatisierungen der Bauchdecke und der Gebärmutterwand, aber auch zu Verletzungen des Fötus, der Plazenta und der Nabelschnur führen könnte.

Das Ankerstück wird erst nach einem Erreichen einer Endposition in der Gebärmutter durch ein Betätigen des Betätigungsmechanismus freigegeben.

Um den Gebärmutterraum zu vergrößern, wird die Gebärmutterwand gedehnt, was zu der Vergrößerung der Gebärmutter führt. Mit Hilfe des sich in der Gebärmutter befindlichen Ankerstücks und des mit dem Ankerstück verbundenen Fadens, der bis zur Außenseite reicht, werden die Gebärmutterwand und die Bauchdecke zu einer Einheit fest verbunden. Dazu wird über das Ankerstück die Gebärmutterwand nach außen an die Innenseite der Bauchdecke gezogen. Danach kann eine Untersuchung oder ein chirurgischer Eingriff an der vergrößerten Gebärmutter der Schwangeren durchgeführt werden.

Die schaftartige Ausgestaltung der beiden Elemente, die das Ankerstück halten, erlauben eine Einführung der Vorrichtung durch einen Trokar, einem Standardinstrument der minimal-invasiven Chirurgie.

Durch eine Anordnung der Backen am distalen Ende des Außenrohrs und am proximalen Ende der Spitze wird die Aussparung, in der das zumindest eine Ankerstück haltbar ist, an beiden Seiten durch die Backen begrenzt. Die Backen dienen als Orientierungsmerkmal beim Einbringen des Ankerelements und erlauben eine visuelle Kontrolle des exakten Sitzes des Ankerelementes.

In einer weiteren Ausgestaltung der Erfindung sind die schaftartigen Elemente derart ausgebildet und angeordnet, dass eine Aussparung gebildet ist, in der das zumindest eine Ankerstück haltbar ist.

Diese Maßnahme hat den Vorteil, dass für eine Aufnahmemöglichkeit eines Ankerstücks in der zwischen den zwei schaftartigen Elementen gebildeten Aussparung gesorgt ist. Dies erlaubt eine schlanke, wenig raumergreifende Bauweise der Vorrichtung.

In einer weiteren Ausgestaltung der Erfindung weist der Betätigungsmechanismus ein Betätigungselement zum Bewegen zumindest eines der schaftartigen Elemente auf, wobei in einer ersten Position der schaftartigen Elemente das zumindest eine Ankerstück in der Aussparung unverlierbar gehalten ist und in einer zweiten Position der schaftartigen Elemente das zumindest eine Ankerstück einlegbar oder freigebbar ist.

Diese Maßnahme hat den Vorteil, dass durch ein einfaches Betätigen des Betätigungselements die schaftartigen Elemente in die erste Position oder in die zweite Position gebracht werden können.

In einer weiteren Ausgestaltung der Erfindung weist der Betätigungsmechanismus eine Feder auf, die das zumindest eine schaftartige Element derart beaufschlagt, dass die schaftartigen Elemente in die erste Position gebracht werden.

Diese Maßnahme hat den Vorteil, dass die schaftartigen Elemente dauernd in Richtung der ersten Position, d.h. in die Position, in der das zumindest eine Ankerstück zwischen den schaftartigen Elementen unverlierbar gehalten ist, mit Federkraft beaufschlagt werden. Somit wird erreicht, dass das zumindest eine Ankerstück zwischen den federbelasteten, schaftartigen Elementen dauernd unverlierbar gehalten wird.

In einer weiteren Ausgestaltung der Erfindung bewegt das Betätigungselement beim Betätigen gegen die Kraft der Feder die schaftartigen Elemente derart, dass diese in die zweite Position gebracht werden, und dass beim Freigeben des Betätigungselements die schaftartigen Elemente wieder in die erste Position gebracht werden.

Diese Maßnahme hat den Vorteil, dass dadurch, dass das zumindest eine schaftartige Element federbelastet ist, die schaftartigen Elemente, durch Loslassen des Betätigungselements automatisch, von der zweiten Position in die erste Position gebracht werden. Dies vereinfacht die Handhabung der Vorrichtung, denn ohne eine Betätigung ist das Ankerelement dauernd fest gehalten.

In einer weiteren Ausgestaltung der Erfindung ist an einem proximalen Ende der schaftartigen Elemente ein Kopf angeordnet.

Diese Maßnahme hat den Vorteil, dass die Vorrichtung an dem Kopf, der sich am proximalen Ende der schaftartigen Elemente befindet und somit während der Untersuchung oder des chirurgischen Eingriffs außerhalb des Körpers der Patientin verbleibt, durch eine menschliche Hand sicher und fest ergriffen werden kann.

In einer weiteren Ausgestaltung der Erfindung ist das Betätigungselement als ein Druckknopf ausgebildet, der an dem Kopf angeordnet ist.

Diese Maßnahme hat den Vorteil, dass das als Druckknopf ausgebildete Betätigungselement von einem Finger, beispielsweise von einem Daumen der Hand, die die Vorrichtung hält, betätigt werden kann. Somit kann die erfindungsgemäße Vorrichtung nur mit einer Hand sowohl gehalten als auch betätigt werden.

In einer weiteren Ausgestaltung der Erfindung ist das Betätigungselement in Richtung der axialen Bewegung der schaftartigen Elemente bewegbar.

Auch diese Ausgestaltung führt zu einer ergonomischen Handhabung der erfindungsgemäßen Vorrichtung in Ein-Hand-Bedienung.

In einer weiteren Ausgestaltung der Erfindung wird der Innenschaft mittels der Feder in Richtung des Kopfs gedrückt.

Diese Maßnahme hat den Vorteil, dass der federbelastete Innenschaft, der in Richtung des Kopfs gedrückt wird, für einen sicheren Sitz des zumindest einen Ankerstücks sorgt, das zwischen dem Innenschaft und dem Außenrohr gehalten ist. Dadurch ist ausgeschlossen, falls der Innenschaft irgendwo anstößt, dieser versehentlich so bewegt wird, dass das Ankerstück freigegeben wird.

In einer weiteren Ausgestaltung der Erfindung steht der Innenschaft mit dem Betätigungsmechanismus derart in Wirkverbindung, dass ein Betätigen des Betätigungselements ein axiales Verschieben des Innenschafts längs einer Längsachse bewirkt.

Diese Maßnahme hat den Vorteil, dass auf eine mechanisch einfache Weise das Betätigen des als der Druckknopf ausgebildeten Betätigungselements in eine axiale Verschiebung des Innenschafts umgesetzt werden kann.

In einer weiteren Ausgestaltung der Erfindung weist jede Backe zumindest eine Frästasche auf.

Diese Maßnahme hat den Vorteil, dass durch eine derartige Ausgestaltung der Erfindung das zumindest eine Ankerstück in den Frästaschen der Backen derart festgehalten ist, dass ein radiales Verschieben des Ankerstücks ausgeschlossen ist.

In einer weiteren Ausgestaltung der Erfindung ist die Aussparung derart ausgebildet, dass in dieser zwei radial gegenüberliegende Ankerstücke haltbar sind.

Diese Maßnahme hat den Vorteil, dass gleichzeitig zwei Ankerstücke in die Gebärmutter eingeführt werden können. Dies ermöglicht das Erweitern und Fixieren der Gebärmutterwand an zwei unterschiedlichen Stellen an der Bauchdecke.

In einer weiteren Ausgestaltung der Erfindung ist an dem Kopf ein Mechanismus zum Anbringen eines Trokars angeordnet.

Diese Maßnahme hat den Vorteil, dass an der erfindungsgemäßen Vorrichtung ein Trokar fixiert werden kann, mit dessen Hilfe eine Öffnung in der Bauchdecke und in der Gebärmutterwand geschaffen werden kann und durch den die Vorrichtung in den Körper eingeführt werden kann.

In einer weiteren Ausgestaltung der Erfindung ist ein Auswurfmechanismus zum Auswerfen des zumindest einen Ankerstücks vorhanden.

Diese Maßnahme hat den Vorteil, dass bei einem Verklemmen oder einem Blockieren des Ankerstücks durch umgebendes Gewebe der Auswurfmechanismus das Lösen des Ankerstücks von der erfindungsgemäßen Vorrichtung sicherstellt.

In einer weiteren Ausgestaltung der Erfindung ist mit der Vorrichtung ein Ankerstück zum Verwenden mit dieser Vorrichtung vorgesehen, das einen Körper aufweist, der in Form eines Streifens ausgebildet ist.

Diese Maßnahme hat den Vorteil, dass das erfindungsgemäße Ankerstück an die dieses haltenden Schäfte eng anlegbar ist.

In einer weiteren Ausgestaltung der Erfindung weist der streifenförmig ausgebildete Körper des Ankerstücks die Geometrie des Abschnitts eines Außenrohrs auf.

Diese Maßnahme hat den Vorteil, dass das erfindungsgemäße Ankerstück nicht radial vor dem Außenrohr vörsteht, sondern vollständig in der Aussparung aufgenommen werden kann. Somit kann die erfindungsgemäße Vorrichtung mit dem zumindest einen Ankerstück in einen Schaft des Trokars eingeführt werden.

In einer weiteren Ausgestaltung der Erfindung ist das Ankerstück an beiden Enden rund ausgebildet.

Diese Maßnahme hat den Vorteil, dass durch eine derartige Ausbildung des Ankerstücks eine Traumatisierung der Gebärmutterwand durch scharfe Ecken oder Kanten ausgeschlossen ist.

In einer weiteren Ausgestaltung der Erfindung ist das Ankerstück etwa mittig mit zumindest einer Öffnung zum Einfädeln eines Zugfadens versehen.

Diese Maßnahme hat den Vorteil, dass mit dem Zugfaden, der aus der Bauchwand nach außen geführt wird, das Ankerstück einfach manipuliert, d.h. zunächst an die Innenseite der Gebärmutter angelegt werden kann. Nach Heranziehen der Gebärmutterwand wird die erweiterte Gebärmutter an der Bauchdecke der Patientin fixiert. Dazu wird an dem von der Außenseite des Körpers abstehenden Ende des Zugfadens gezogen und dieser wird dann, beispielsweise durch eine Klemme, am Körper fixiert. Somit bilden die Gebärmutterwand und die Bauchdecke eine fest verbundene Einheit.

In einer weiteren Ausgestaltung der Erfindung ist das Ankerstück mit zumindest einer endseitigen Öffnung zum Einfädeln eines Bergefadens versehen.

Diese Maßnahme hat den Vorteil, dass mit Hilfe des Bergefadens, der sich im Inneren der Gebärmutter befindet, das Ankerstück nach der Untersuchung oder des chirurgischen Eingriffs wieder aus dem Körper entfernt werden kann.

In einer weiteren Ausgestaltung der Erfindung sind der Zugfaden und der Bergefaden des an der Vorrichtung unverlierbar gehaltenen Ankerstücks im Inneren eines Schaftes eines Trokars anordenbar.

Diese Maßnahme hat den Vorteil, dass durch die Anordnung der beiden Fäden in dem Trokarschaft die Fäden das Einführen der Vorrichtung durch die Bauchdecke in die Gebärmutter nicht beeinträchtigen und auch selbst dabei nicht beeinträchtigt werden.

In einer weiteren Ausgestaltung der Erfindung sind der Zugfaden und der Bergefaden des an der Vorrichtung unverlierbar gehaltenen Ankerstücks außerhalb des Schaftes eines Trokars anordenbar.

Diese Maßnahme hat den Vorteil, dass wenn die Fäden aufgrund der Größe des vorhandenen Trokars nicht in den Trokarschaft passen, sie beim Einführen der Vorrichtung in die Gebärmutter außerhalb des Trokarschaftes angeordnet werden können.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:
- Figur 1: eine perspektivische Seitenansicht einer erfindungsgemäßen Vorrichtung,
- Figur 2: eine vergrößere Darstellung eines Ankerstücks von Figur 1,
- Figur 3: einen Schnitt entlang einer Längsachse von Figur 1, wobei kein Ankerstück zwischen den schaftartigen Elementen gehalten ist,
- Figur 4: eine vergrößerte perspektivische Seitenansicht der erfindungsgemäßen Vorrichtung vor einem Einlegen des Ankerstücks,
- Figur 5: eine der Darstellung von Figur 4 vergleichbare Darstellung, wobei das Ankerstück zwischen den schaftartigen Elementen gehalten ist,
- Figur 6: ein weiteres Ausführungsbeispiel in einer der Darstellung von Figur 4 vergleichbaren Darstellung, bei dem zwei Ankerstücke einlegbar sind,
- Figur 7: eine Seitenansicht einer erfindungsgemäßen Vorrichtung, an der ein Trokar angebracht ist, wobei die Vorrichtung teilweise in die Trokarhülse eingeschoben ist.
- Figur 8: einen Schnitt durch einen Unterleib einer schwangeren Patientin im Bereich der Vagina und der Gebärmutter, wobei die erfindungsgemäße Vorrichtung in die Gebärmutter durch die Bauchdecke eingeführt ist und das Ankerstück von der erfindungsgemäßen Vorrichtung freigegeben worden ist, und
- Figur 9: eine der Schnittdarstellung von Figur 8 vergleichbare Schnittdarstellung, wobei an einer Seite der Gebärmutter die Gebärmutterwand an der Bauchdecke fixiert ist und an der anderen Seite der Gebärmutter ein Bergen eines Ankerstücks dargestellt ist.

Eine in den Figuren dargestellte Vorrichtung zum Einführen zumindest eines Ankerstücks in einen Hohlraum eines Lebewesens ist in ihrer Gesamtheit mit der Bezugsziffer 10 bezeichnet.

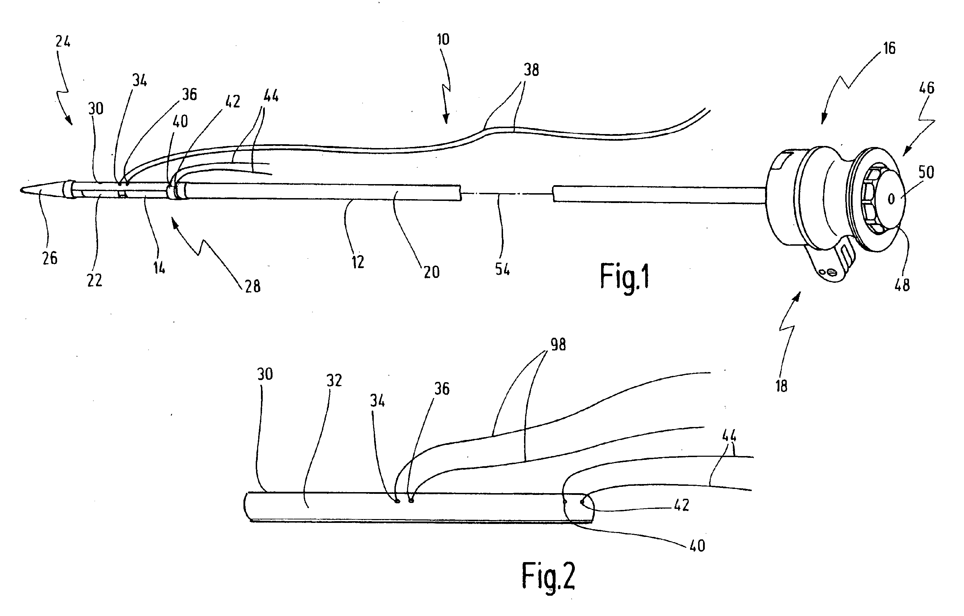

Wie insbesondere aus Figur 1 hervorgeht, weist die erfindungsgemäße Vorrichtung 10 zwei schaftartige Elemente 12, 14 auf, an deren proximalen Ende 16 ein Kopf 18 angeordnet ist. Die schaftartigen Elemente 12, 14 sind relativ zueinander bewegbar.

In diesem Ausführungsbeispiel ist eines der schaftartigen Elemente 12 als ein Außenrohr 20 ausgebildet. Das andere schaftartige Element 14 ist dagegen als ein in dem Außenrohr 20 aufgenommener Innenschaft 22 ausgebildet.

Ein distales Ende 24 des Innenschaftes 22 ist als eine Spitze 26 ausgebildet, die ein Anzielen und ein Einführen der erfindungsgemäßen Vorrichtung 10 durch die Bauchdecke in die Gebärmutter vereinfacht.

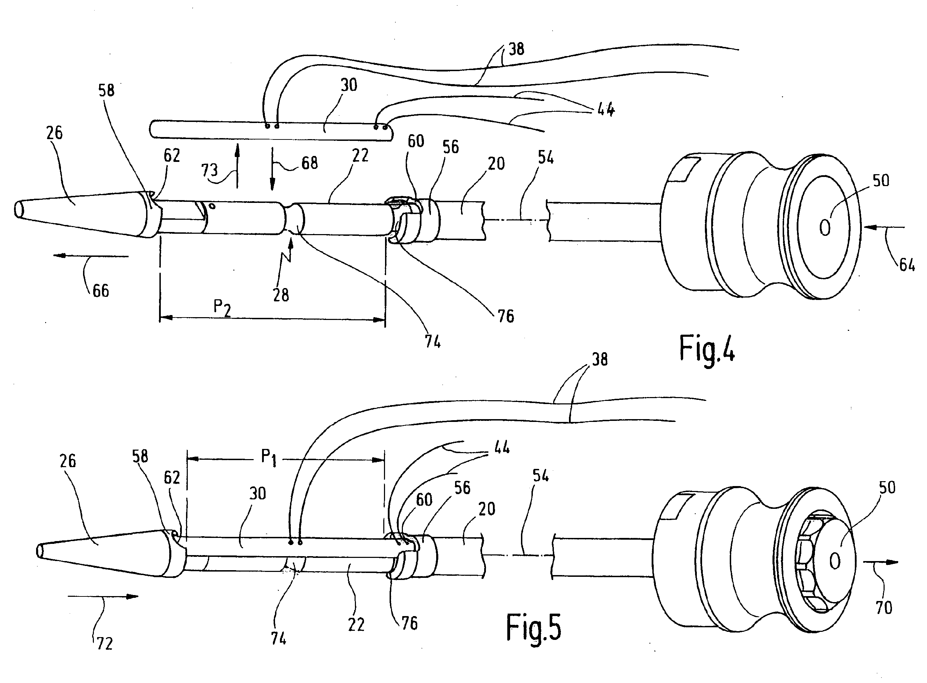

Zwischen dem distalen Ende des Außenrohrs 20 und dem distalen Ende des Innenschafts 22 ist eine Aussparung 28 ausgebildet, wie dies insbesondere aus der vergrö-ßerten Darstellung von Figur 4 ersichtlich ist.

In der Aussparung 28 kann ein Ankerstück 30 aufgenommen werden.

Das Ankerstück 30 weist einen streifenförmigen Körper 32 auf (siehe auch Figur 2).

Der Körper 32 ist in Form eines Rohrabschnitts ausgebildet, dessen Geometrie der Geometrie des Außenrohrs 20 entspricht. Das Ankerstück 30 ist an seinen beiden Enden abgerundet ausgebildet.

Das Ankerstück 30 weist zwei mittige Öffnungen 34, 36 auf, durch die ein Zugfaden 38 gefädelt ist. Zusätzlich weist das Ankerstück 30 zwei endseitige Öffnungen 40, 42 auf, die an einer Seite des Ankerstücks 30 angeordnet sind. Durch die endseitigen Öffnungen 40, 42 ist ein Bergefaden 44 gefädelt.

Der Zugfaden 38, der deutlich länger ist als der Bergefaden 44, dient dazu, die Gebärmutterwand an der Bauchdecke zu fixieren. Der kürzere Bergefaden 44 dagegen dient dazu, das Ankerstück 30 am Ende der Untersuchung oder des chirurgischen Eingriffs zu entfernen, wie dies nachfolgend noch beschrieben wird.

Der Innenschaft 22 weist zwei Vertiefungen 74, 76 auf, die an der Stelle angeordnet sind, an der die mittigen Öffnungen 34, 36 und die endseitigen Öffnungen 40, 42 des Ankerstücks 30 zum Liegen kommen. In diesen Vertiefungen 74, 76 können die Fäden 38, 44 eingelegt werden.

Die erfindungsgemäße Vorrichtung 10 weist einen Betätigungsmechanismus 46 auf, über den eines der schaftartigen Elemente 12, 14 derart bewegbar ist, dass das von diesen gehaltene Ankerstück 30 freigegeben wird.

Der Betätigungsmechanismus 46 weist ein Betätigungselement 48 auf, das an dem Kopf 18 angeordnet ist.

In diesem Ausführungsbeispiel ist das Betätigungselement 48 als ein Druckknopf 50 ausgebildet, der in Richtung der axialen Bewegung der schaftartigen Elemente 12, 14 bewegbar ist.

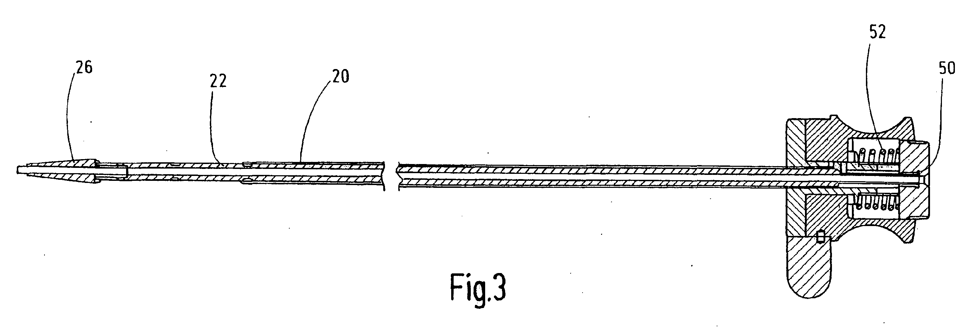

Der Betätigungsmechanismus 46 weist eine Feder 52 auf, wie es aus dem Schnitt längs einer Längsachse 54 von Figur 3 ersichtlich ist.

Die Feder 52 beaufschlagt den Innenschaft 22, und zwar derart, dass der Innenschaft 22 mittels der Feder 52 in Richtung des Kopfs 18 gedrückt wird. Beim Drücken des Druckknopfes 50 wird der Innenschaft 22 nach distal verschoben.

Aus der vergrößerten perspektivischen Seitenansicht von Figuren 4 und 5 ist ersichtlich, dass an dem distalen Ende des Außenrohrs 20 und an dem proximalen Ende der an dem distalen Ende des Innenschaftes 22 ausgebildeten Spitze 26 jeweils eine Backe 56, 58 ausgebildet ist. Jede Backe 56, 58 weist jeweils eine Frästasche 60, 62 auf. Die an dem distalen Ende des Außenrohrs 20 ausgebildete Frästasche 60 liegt genau gegenüber der an dem proximalen Ende der Spitze 26 ausgebildeten Frästasche 62. Die gegenüberliegenden Frästaschen 60, 62 vermeiden ein radiales Verschieben des Ankerstücks 30, das zwischen den Backen 56, 58 haltbar ist. Die Form der Frästaschen entspricht der Rundung der abgerundeten Enden des Ankerstücks 30.

Das Betätigen des als der Druckknopf 50 ausgebildeten Betätigungselements 48, was in diesem Ausführungsbeispiel durch ein Drücken des Druckknopfs 50 erfolgt und mit einem Pfeil 64 in Figur 4 angezeigt ist, bewirkt ein axiales Verschieben des Innenschafts 22 längs der Längsachse 54. Das axiale Verschieben ist mit einem Pfeil 66 angezeigt.

Durch das axiale Verschieben des Innenschaftes 22 werden die beiden schaftartigen Elemente 12, 14, nämlich das Außenrohr 20 und der Innenschaft 22, in eine zweite Position P₂ gebracht. Die zweite Position ist als ein Abstand zwischen den beiden Backen 56, 58 definiert, der größer ist als die Länge des Ankerstücks 30.

In der zweiten Position P₂ ist das Ankerstück 30 in die zwischen den Backen 56, 58 ausgebildete Aussparung 28 einlegbar. Das Einlegen des Ankerstücks 30 ist mit einem Pfeil 68 angezeigt.

Nachdem das Ankerstück 30 in der Aussparung 28 eingelegt worden ist, wird der mit der Feder 52 (siehe Figur 3) federbelastete Innenschaft 22 nach Freigeben des Druckknopfs 50 (Pfeil 70, Figur 5) in Richtung des proximalen Endes 16 der Vorrichtung 10 automatisch bewegt. Die proximale Bewegung des Innenschaftes 22 ist in Figur 5 mit einem Pfeil 72 angezeigt.

Dadurch werden die schaftartigen Elemente 12, 14 in eine erste Position P₁ gebracht. In dieser ist das Ankerelement 30 unverlierbar zwischen den Backen 56, 58 gehalten.

Um das Ankerstück 30 wieder freizugeben, müssen die zwei schaftartigen Elemente 12, 14 durch das Drücken des Druckknopfes 50 (siehe Pfeil 64, Figur 4) in die zweite Position P₂ gebracht werden. Das Freigeben des Ankerstücks 30 ist mit einem Pfeil 73 in Figur 4 angezeigt.

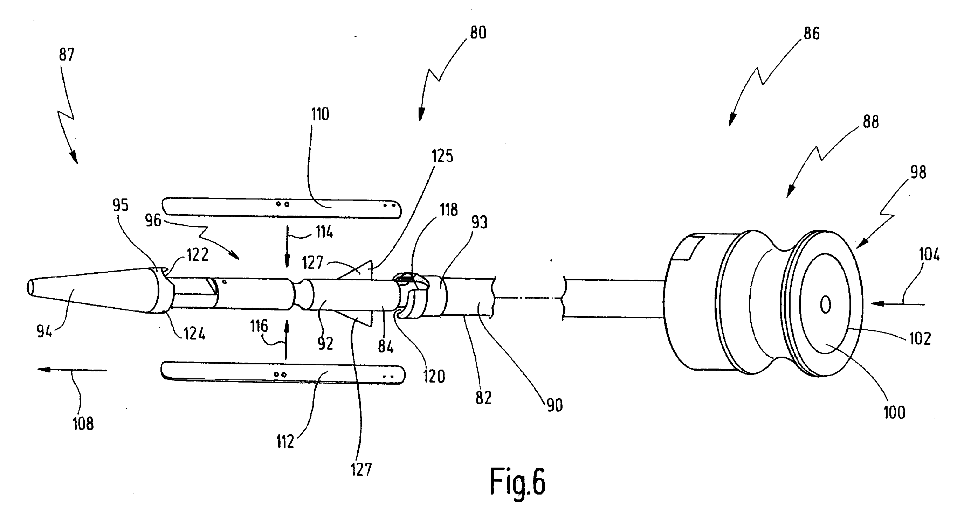

In Figur 6 ist ein Ausführungsbeispiel 80 der erfindungsgemäßen Vorrichtung dargestellt, das sich von dem in Figur 4 dargestellten Ausführungsbeispiel hinsichtlich der Zahl der Ankerstücke, die zwischen den schaftartigen Elementen haltbar sind, unterscheidet.

Bei dem in Figur 6 dargestellten Ausführungsbeispiel weist die Vorrichtung 80 zwei ebenfalls schaftartige Elemente 82, 84 auf, an deren proximalen Ende 86 ein Kopf 88 angeordnet ist.

Eines der schaftartigen Elemente 82 ist ebenfalls als ein Außenrohr 90 und das andere schaftartige Element 84 als ein in dem Außenrohr 90 aufgenommener, axial bewegbarer Innenschaft 92 ausgebildet.

Ein distales Ende 87 des Innenschaftes 92 ist ebenfalls als eine Spitze 94 ausgebildet.

Zwischen dem distalen Ende des Außenrohrs 90 und dem distalen Ende des Innenschafts 92 ist ebenfalls eine Aussparung 96 ausgebildet.

An dem distalen Ende des Außenrohrs 90 und an dem proximalen Ende der Spitze 94 ist jeweils eine Backe 93, 95 angeordnet.

Die Vorrichtung 80 weist ebenfalls einen Betätigungsmechanismus 98 auf, der ebenfalls ein als ein Druckknopf 100 ausgebildetes Betätigungselement 102 aufweist.

Im Vergleich zu dem Ausführungsbeispiel von Figur 4 ist zu erkennen, dass in der Vorrichtung 80 zwei gegenüberliegende Ankerstücke 110, 112 einlegbar sind.

Im Unterschied zu dem Ausführungsbeispiel von Figur 4 weist jede Backe 93, 95 zwei gegenüberliegende Frästaschen 118, 120; 122, 124 auf.

Der Betätigungsmechanismus 98 wird gleich wie bei dem ersten in der Figur 4 dargestellten Ausführungsbeispiel betätigt.

Ein Drücken des Druckknopfs 100, was mit einem Pfeil 104 angezeigt ist, bewirkt ein axiales Verschieben längs einer Längsachse 106 des Innenschafts 92 (siehe Pfeil 108). Somit werden die schaftartigen Elemente 82, 84 in die zweite Position P₂ gebracht.

In der zweiten Position P₂ werden die zwei Ankerstücke 110, 112 in die Aussparung 96 eingelegt. Das Einlegen der beiden Ankerstücke 110, 112 ist mit Pfeilen 114, 116 angezeigt.

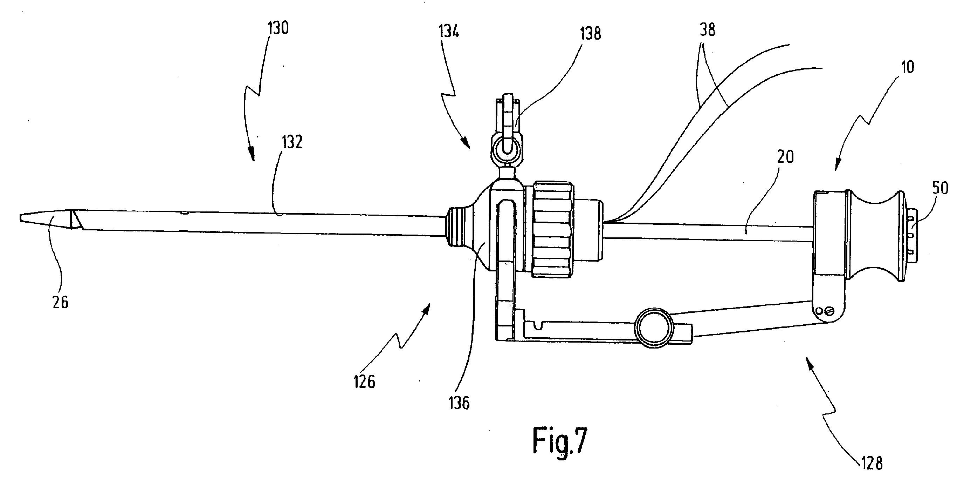

In Figur 7 ist eine Seitenansicht der Vorrichtung 10 dargestellt, an der ein Trokar 126 mittels eines Mechanismus 128 fixiert ist. Trokare dienen dazu, medizinische Instrumente von der Außenseite in den Körper durch eine Inzision einzuführen.

Der Trokar weist eine Trokarhülse 130 auf, die einen Schaft 132 aufweist, an dessen proximalseitigem Ende ein Gehäuse 134 angeordnet ist. Das Gehäuse 134 weist einen äußeren etwa hohlzylindrischen Abschnitt 136 auf, der sich unmittelbar an das proximale Ende des hohlen Schaftes 132 anschließt. Vom äußeren Abschnitt 136 steht radial ein Anschluss 138 vor. Über den Anschluss 138 kann ein Gas in den Innenraum des Gehäuses 134 und durch den Schaft 132 hindurchgeführt werden.

In diesem in Figur 7 dargestellten Ausführungsbeispiel ist der Zugfaden 38 und der hier nicht ersichtliche Bergefaden 44 im Inneren des Schaftes 132 des Trokars 126 angeordnet.

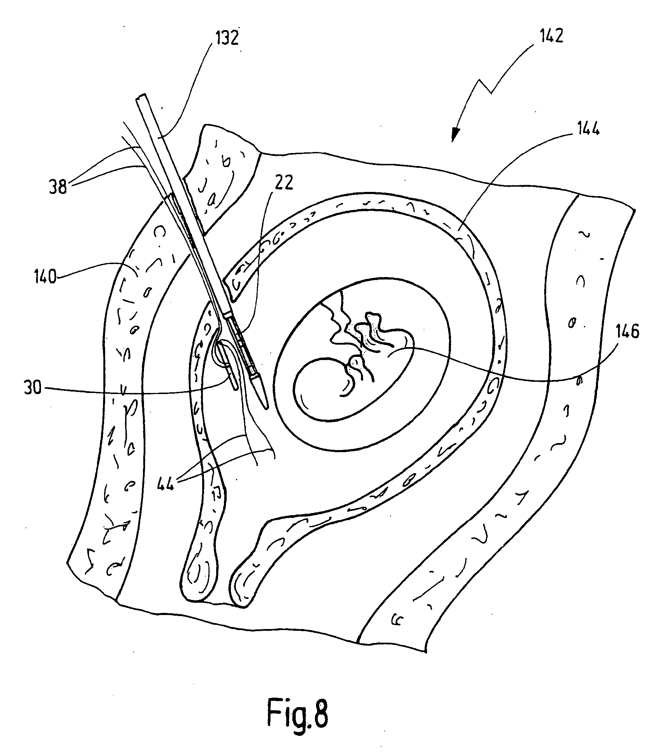

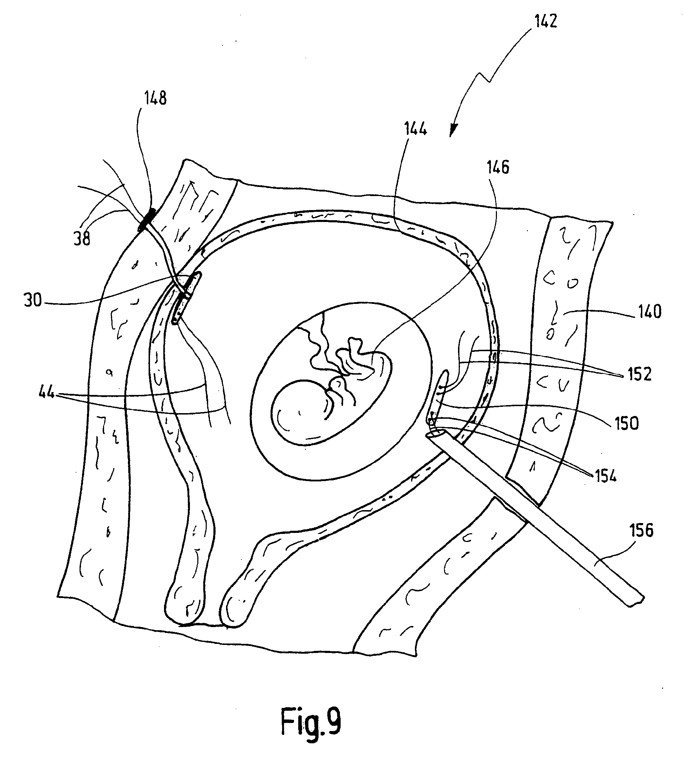

Der Einsatz der erfindungsgemäßen Vorrichtung 10 soll im Ablauf der Figuren 8 und 9 kurz erläutert werden.

Die schaftartigen Elemente samt eingelegtem Ankerstück (oder -stücken) werden direkt oder über einen Trokar durch die Bauchdecke 140 und die Gebärmutterwand 144 in den Innenraum der Gebärmutter 142 eingebracht. Dies kann unter endoskopischer Beobachtung erfolgen.

In Figur 8 ist eine Situation dargestellt, in der das Ankerstück 30 bereits freigegeben worden ist.

Aus dieser Figur ist ersichtlich, dass der Zugfaden 38 des sich in der Gebärmutter 142 befindlichen Ankerstücks 30 aus der Bauchdecke 140 der Patientin nach außen geführt ist. Der Bergefaden 44 dagegen befindet sich in der Gebärmutter 142.

Danach wird die Vorrichtung 10 aus der Gebärmutter 142 entfernt und die Gebärmutterwand 144 wird mittels des sich in der Gebärmutter 142 befindlichen Ankerstücks 30 und des durch das Ankerstück 30 gefädelten Zugfadens 38 an die Innenseite der Bauchdecke 140 herangezogen und der Zugfaden wird von der Außenseite der Bauchdecke 140 mit einer Klemme 148 fixiert (Figur 9, links).

Aus der Darstellung von Figur 9 ist ersichtlich, dass das Heranziehen der Gebärmutterwand 144 an die Bauchdecke 140 zur Vergrößerung der Gebärmutter 142 führt.

Nach dem Fixieren kann eine hier nicht dargestellte pränatale Untersuchung oder ein chirurgischer Eingriff an dem Fötus 146 oder an der Gebärmutter 142 mit Hilfe medizinischer Instrumente durchgeführt werden.

Nach der durchgeführten Untersuchung oder dem durchgeführten chirurgischen Eingriff wird der Zugfaden 152 abgeschnitten und es wird ein medizinisches Instrument 156 durch die Bauchdecke 140 in die Gebärmutter 142 eingeführt, mittels dem der sich im Inneren der Gebärmutter 142 befindliche Bergefaden 154 des Ankerstückes 150 ergriffen wird. Das Ankerstück 150 wird durch einen Schaft des medizinisches Instruments 156 von der Gebärmutter 142 entfernt (Figur 9, rechts). Dieses Einziehen in den Schaft wird dadurch erleichtert, dass der Bergefaden 154 endseitig am Ankerstück 130 angebracht ist.

Wie aus Figur 6 zu entnehmen weist der Innenschaft 92 einen Auswurfmechanismus 125 auf. Dieser Auswurfmechanismus 125 ermöglicht das seitliche Auswerfen der Ankerstücke 110 und 112 von dem Innenschaft 92, falls die Ankerstücke 110, 112 durch Gewebe oder Gewebeflüssigkeiten vor seitlichem Abfallen gehindert sind. Im gezeigten Fall weist der Auswurfmechanismus zwei seitlich ausfahrbare, federbelastete Nasen 127 auf. Ein solcher Auswurfmechanismus kann auch bei der Ausführung von Figuren 1 bis 5 vorgesehen sein.

## Patentansprüche

1. Vorrichtung zum Einführen zumindest eines Ankerstücks in einen Hohlraum eines Lebewesens, um eine Wand des Hohlraumes mittels des zumindest einen Ankerstücks zeitweilig an einer Körperwand zu fixieren, mit zwei relativ zueinander axial bewegbaren, schaftartigen Elementen (12, 14; 82, 84), zwischen denen das zumindest eine Ankerstück (30; 110, 112) unverlierbar haltbar ist, wobei eines der schaftartigen Elemente (12, 14; 82, 84) als ein Außenrohr (20, 40) und das andere als ein in dem Außenrohr (20, 90) aufgenommener, axial verschiebbarer Innenschaft (22, 92) ausgebildet ist, dessen distales Ende als eine Spitze (26, 94) ausgebildet ist, und mit einem Betätigungsmechanismus (46, 98), über den zumindest ein Element (14, 84) derart bewegbar ist, dass das gehaltene Ankerstück (30; 110, 112) freigebbar ist, **dadurch gekennzeichnet, dass** das zumindest eine Ankerstück (30; 110, 112) an axial gegenüberliegenden Enden unverlierbar haltbar ist, dass die schaftartigen Elemente (12, 14; 82, 84) jeweils eine Backe (56, 58; 93, 95) aufweisen, zwischen denen das zumindest eine Ankerstück (30; 110, 112) unverlierbar haltbar ist und dass eine Backe (56, 93) an dem distalen Ende des Außenrohrs (20, 90) und die andere Backe (58, 95) an dem proximalen Ende der Spitze (26, 94) angeordnet ist.

2. Vorrichtung nach Anspruch 1, **dadurch gekennzeichnet, dass** die schaftartigen Elemente (12, 14; 82, 84) derart ausgebildet und angeordnet sind, dass eine Aussparung (28, 96) gebildet ist, in der das zumindest eine Ankerstück (30; 110, 112) haltbar ist.

3. Vorrichtung nach Anspruch 2, **dadurch gekennzeichnet, dass** der Betätigungsmechanismus (46, 98) ein Betätigungselement (48, 102) zum Bewegen zumindest eines der schaftartigen Elemente (14, 84) aufweist, wobei in einer ersten Position der schaftartigen Elemente (12, 14; 82, 84) das zumindest eine Ankerstück (30; 110, 112) in der Aussparung (28, 96) unverlierbar gehalten ist und in einer zweiten Position der schaftartigen Elemente (12, 14; 82, 84) das zumindest eine Ankerstück (30; 110, 112) einlegbar oder freigebbar ist.

4. Vorrichtung nach einem der Ansprüche 1 bis 3, **dadurch gekennzeichnet, dass** der Betätigungsmechanismus (46, 98) eine Feder (52) aufweist, die das zumindest eine schaftartige Element (14, 84) derart beaufschlagt, dass die schaftartigen Elemente (12, 14; 82, 84) in die erste Position gebracht werden.

5. Vorrichtung nach Anspruch 4, **dadurch gekennzeichnet, dass** das Betätigungselement (48, 102) beim Betätigen gegen die Kraft der Feder (52) die schaftartigen Elemente (12, 14; 82, 84) derart bewegt, dass diese in die zweite Position gebracht werden und dass beim Freigeben des Betätigungselements (48, 102) die schaftartigen Elemente (12, 14; 82, 84) wieder in die erste Position gebracht werden.

6. Vorrichtung nach einem der Ansprüche 1 bis 5, **dadurch gekennzeichnet, dass** an einem proximalen Ende (16, 86) der schaftartigen Elemente (12, 14; 82, 84) ein Kopf (18, 88) angeordnet ist.

7. Vorrichtung nach Anspruch 6, **dadurch gekennzeichnet, dass** das Betätigungselement (48, 102) als ein Druckknopf (50, 100) ausgebildet ist, der an dem Kopf (18, 88) angeordnet ist.

8. Vorrichtung nach einem der Ansprüche 3 bis 7, **dadurch gekennzeichnet, dass** das Betätigungselement (48, 102) in Richtung der axialen Bewegung der schaftartigen Elemente (12, 14; 82, 84) bewegbar ist.

9. Vorrichtung nach einem der Ansprüche 1 bis 8, **dadurch gekennzeichnet, dass** der Innenschaft (22, 92) mittels einer Feder (52) in Richtung des Kopfs (18, 88) gedrückt wird.

10. Vorrichtung nach einem der Ansprüche 1 bis 9, **dadurch gekennzeichnet, dass** der Innenschaft (22, 92) mit dem Betätigungsmechanismus (46, 98) derart in Wirkverbindung steht, dass ein Betätigen des Betätigungselements (48, 102) ein axiales Verschieben des Innenschafts (22, 92) längs einer Längsachse (54, 106) bewirkt.

11. Vorrichtung nach einem der Ansprüche 1 bis 10, **dadurch gekennzeichnet, dass** jede Backe (56, 58; 93, 95) zumindest eine Frästasche (60, 62; 118, 120, 122, 124) aufweist.

12. Vorrichtung nach einem der Ansprüche 2 bis 11, **dadurch gekennzeichnet, dass** die Aussparung (28, 96) derart ausgebildet ist, dass in dieser zwei gegenüberliegende Ankerstücke (110, 112) haltbar sind.

13. Vorrichtung nach einem der Ansprüche 1 bis 12, **dadurch gekennzeichnet, dass** jede Backe (93, 95) zwei gegenüberliegende Frästaschen (118, 120, 122, 124) aufweist.

14. Vorrichtung nach einem der Ansprüche 1 bis 13, **dadurch gekennzeichnet, dass** an dem Kopf (18, 88) ein Mechanismus (128) zum Anbringen eines Trokars (126) angeordnet ist.

15. Vorrichtung nach einem der Ansprüche 1 bis 14, **dadurch gekennzeichnet, dass** ein Auswurfmechanismus (125) zum Auswerfen des zumindest einen Ankerstücks (110, 112) vorhanden ist.

16. Vorrichtung nach einem der Ansprüche 1 bis 15 mit einem Ankerstück zum Verwenden mit dieser Vorrichtung, das einen Körper (32) aufweist, der streifenförmig ausgebildet ist.

17. Vorrichtung mit einem Ankerstück nach Anspruch 16, **dadurch gekennzeichnet, dass** der streifenförmige Körper als Rohrabschnitt ausgebildet ist und eine Geometrie des Außenrohrs (20, 90) aufweist.

18. Vorrichtung mit einem Ankerstück nach Anspruch 16 oder 17, **dadurch gekennzeichnet, dass** das Ankerstück (30; 110, 112) an beiden Enden rund ausgebildet ist.

19. Vorrichtung mit einem Ankerstück nach einem der Ansprüche 16 bis 18, **dadurch gekennzeichnet, dass** das Ankerstück (30; 110, 112) mit zumindest einer etwa mittigen Öffnung (34, 36) zum Einfädeln eines Zugfadens (38) versehen ist.

20. Vorrichtung mit einem Ankerstück nach einem der Ansprüche 16 bis 19, **dadurch gekennzeichnet, dass** das Ankerstück (30; 110, 112) mit zumindest einer endseitigen Öffnung (40, 42) zum Einfädeln eines Bergefadens (44) versehen ist.

## Claims

1. Device for inserting at least one anchor piece into a hollow space of a living being, in order to fix a wall of the hollow space temporarily to a wall of the body by means of the at least one anchor piece, with two shaft-like elements (12, 14; 82, 84) which are axially movable relative to one another and by which the at least one anchor piece (30; 110, 112) can be held captive, wherein one of the shaft-like elements (12, 14; 82, 84) is designed as an outer tube (20, 90), and the other is designed as an axially displaceable inner shaft (22, 92) received in the outer tube (20, 90) which distal end is designed as a tip (26, 94), and with an actuating mechanism (46, 98) via which at least one element (14, 84) can be moved in such a way that the anchor piece (30; 110, 112) held by it can be released, **characterized in that** the at least one anchor piece (30; 110, 112) is held captive at axial opposite ends, further **in that** the shaft-like elements (12, 14; 82, 84) each have a jaw (56, 58; 93, 95) between which the at least one anchor piece (30; 110, 112) can be held captive, and **in that** one jaw (56, 53) is arranged on the distal end of the outer tube (20, 90) and the other jaw (58, 95) is arranged on the proximal end of the tip (26, 94).

2. Device of claim 1, **characterized in that** the shaft-like elements (12, 14; 82, 84) are designed and arranged in such a way that a recess (28, 96) is formed in which the at least one anchor piece (30; 110, 112) can be held.

3. Device of claim 2, **characterized in that** the actuating mechanism (46, 98) has an actuating element (48, 102) for moving at least one of the shaft-like elements (14, 84), where, in a first position of the shaft-like elements (12, 14; 82, 84) the at least one anchor piece (30; 110, 112) is held captive in the recess (28, 96), and, in a second position of the shaft-like elements (12, 14; 82, 84), the at least one anchor piece (30; 110, 112) can be fitted or released.

4. Device of any one of claims 1 through 3, **characterized in that** the actuating mechanism (46, 98) has a spring (52), which acts on the at least one shaft-like element (14, 84) in such a way that the shaft-like elements (12, 14; 82, 84) are brought into the first position.

5. Device of claim 4, **characterized in that** the actuating element (48, 102), upon actuation counter to the force of the spring (52), moves the shaft-like elements (12, 14; 82, 84) in such a way that they are brought into the second position, and **in that**, upon release of the actuating element (48, 102), the shaft-like elements (12, 14; 82, 84) are brought back into the first position.

6. Device of any one of claims 1 through 5, **characterized in that** a head (18, 88) is arranged on a proximal end (16, 86) of the shaft-like elements (12, 14; 82, 84).

7. Device of claim 6, **characterized in that** the actuating element (48, 102) is designed as a push-button (50, 100) arranged on the head (18, 88).

8. Device of any one of claims 3 through 7, **characterized in that** the actuating element (48, 102) is movable in the direction of the axial movement of the shaft-like elements (12, 14; 82, 84).

9. Device of any one of claims 1 through 8, **characterized in that** the inner shaft (22, 92) is urged by means of a spring (52) in the direction of the head (18, 88).

10. Device of any one of claims 1 through 9, **characterized in that** the inner shaft (22, 92) is operatively connected to the actuating mechanism (46, 98) in such a way that an actuation of the actuating element (48, 102) causes an axial displacement of the inner shaft (22, 92) along a longitudinal axis (54, 106).

11. Device of any one of claims 1 through 10, **characterized in that** each jaw (56, 58; 93, 95) has at least one pocket (60, 62; 118, 120, 122, 124).

12. Device of any one of claims 2 through 11, **characterized in that** the recess (28, 96) is designed in such a way that two mutually opposite anchor pieces (110, 112) can be held in it

13. Device of any one of claims 1 through 12, **characterized in that** each jaw (93, 95) has two mutually opposite pockets (18, 120, 122, 124).

14. Device of any one of claims 1 through 13, **characterized in that** a mechanism (128) for application of a trocar (126) is arranged on the head (18, 88).

15. Device of any one of claims 1 through 14, **characterized in that** an ejection mechanism (125) is provided for ejecting the at least one anchor piece (110, 112).

16. Device of any one of claims 1 through 15, with an anchor piece for use with the device having a body (32) designed in the shape of a strip.

17. Device with an anchor piece of claim 16, **characterized in that** the strip-like body is designed as a tube section and has a geometry of the outer tube (20, 90).

18. Device with an anchor piece of claim 16 or 17, **characterized in that** the anchor piece (30; 110, 112) is rounded at both ends.

19. Device with an anchor piece of any one of claims 16 through 18, **characterized in that** the anchor piece (30; 110, 112) is provided with at least one approximately central opening (34, 36) for engagement of a draw-thread (38).

20. Device with an anchor piece of any one of claims 16 through 19, **characterized in that** the anchor piece (30; 110, 112) is provided with at least one end opening (40, 42) for engagement of a retrieval thread (44).

## Revendications

1. Dispositif conçu pour insérer au moins une pièce d'ancrage dans une cavité d'un organisme vivant, en vue de bloquer provisoirement à demeure une paroi de ladite cavité sur une paroi corporelle, au moyen de ladite pièce d'ancrage à présence minimale, comprenant deux éléments (12, 14 ; 82, 84) du type tige, mobiles axialement l'un par rapport à l'autre et entre lesquels ladite pièce d'ancrage (30 ; 110, 112), à présence minimale, peut être retenue de manière imperdable, sachant que l'un desdits éléments (12, 14 ; 82, 84) du type tige est réalisé sous la forme d'un tube extérieur (20, 90), l'autre élément étant réalisé sous la forme d'un fût intérieur (22, 92) qui peut coulisser axialement, est logé dans ledit tube extérieur (20, 90), et dont l'extrémité distale revêt la forme d'une pointe (26, 94) ; et un mécanisme d'actionnement (46, 98) par l'intermédiaire duquel au moins un élément (14, 84) peut être mû de telle sorte que la pièce d'ancrage retenue (30 ; 110, 112) puisse être libérée, **caractérisé par le fait que** la pièce d'ancrage (30 ; 110, 112), à présence minimale, peut être retenue de manière imperdable par des extrémités axialement opposées ; **par le fait que** les éléments (12, 14 ; 82, 84) du type tige sont respectivement pourvus de mors (56, 58 ; 93, 95) entre lesquels ladite pièce d'ancrage (30 ; 110, 112), à présence minimale, peut être retenue de manière imperdable ; et **par le fait qu'**un mors (56, 93) est placé à l'extrémité distale du tube extérieur (20, 90), l'autre mors (58, 95) étant placé à l'extrémité proximale de la pointe (26, 94).

2. Dispositif selon la revendication 1, **caractérisé par le fait que** les éléments (12, 14 ; 82, 84), du type tige, sont réalisés et agencés de façon à donner naissance à une zone évidée (28, 96) dans laquelle peut être retenue la pièce d'ancrage (30 ; 110, 112) à présence minimale.

3. Dispositif selon la revendication 2, **caractérisé par le fait que** le mécanisme d'actionnement (46, 98) comporte un élément d'actionnement (48, 102) destiné à mouvoir au moins l'un (14, 84) des éléments du type tige, sachant que, dans un premier emplacement occupé par les éléments (12, 14 ; 82, 84) du type tige, la pièce d'ancrage (30 ; 110, 112) à présence minimale est retenue de manière imperdable dans la zone évidée (28, 96), et que ladite pièce d'ancrage (30 ; 110, 112) à présence minimale peut être insérée ou libérée dans un second emplacement occupé par lesdits éléments (12, 14 ; 82, 84) du type tige.

4. Dispositif selon l'une des revendications 1 à 3, **caractérisé par le fait que** le mécanisme d'actionnement (46, 98) comporte un ressort (52) sollicitant l'élément (14, 84) du type tige, à présence minimale, de façon que les éléments (12, 14 ; 82, 84) du type tige soient amenés au premier emplacement.

5. Dispositif selon la revendication 4, **caractérisé par le fait que**, lors de la manoeuvre, l'élément d'actionnement (48, 102) imprime un mouvement aux éléments (12, 14 ; 82, 84) du type tige, en opposition à la force du ressort (52), de façon telle qu'ils soient amenés au second emplacement ; et **par le fait que** lesdits éléments (12, 14 ; 82, 84) du type tige sont ramenés au premier emplacement lors de la libération dudit élément d'actionnement (48, 102).

6. Dispositif selon l'une des revendications 1 à 5, **caractérisé par le fait qu'**une tête (18, 88) est située à une extrémité proximale (16, 86) des éléments (12, 14 ; 82, 84) du type tige.

7. Dispositif selon la revendication 6, **caractérisé par le fait que** félément d'actionnement (48, 102) est réalisé sous la forme d'un bouton-poussoir (50, 100) placé sur la tête (18, 88).

8. Dispositif selon l'une des revendications 3 à 7, **caractérisé par le fait que** l'élément d'actionnement (48, 102) peut être mû dans la direction du mouvement axial des éléments (12, 14 ; 82, 84) du type tige.

9. Dispositif selon l'une des revendications 1 à 8, **caractérisé par le fait que** le fût intérieur (22, 92) est poussé en direction de la tête (18, 88), au moyen d'un ressort (52).

10. Dispositif selon l'une des revendications 1 à 9, **caractérisé par le fait que** le fût intérieur (22, 92) est en liaison opérante avec le mécanisme d'actionnement (46, 98), de telle sorte qu'une manoeuvre de l'élément d'actionnement (48, 102) provoque un coulissement axial dudit fût intérieur (22, 92) le long d'un axe longitudinal (54, 106).

11. Dispositif selon l'une des revendications 1 à 10, **caractérisé par le fait que** chaque mors (56, 58 ; 93, 95) présente au moins un logement fraisé (60, 62 ; 118, 120, 122, 124).

12. Dispositif selon l'une des revendications 2 à 11, **caractérisé par le fait que** la zone évidée (28, 96) est réalisée de façon telle que deux pièces d'ancrage (110, 112), situées en vis-à-vis, puissent être retenues dans cette dernière.

13. Dispositif selon l'une des revendications 1 à 12, **caractérisé par le fait que** chaque mors (93, 95) comprend deux logements fraisés (118, 120, 122, 124) tournés mutuellement à l'opposé.

14. Dispositif selon l'une des revendications 1 à 13, **caractérisé par le fait qu'**un mécanisme (128) est placé sur la tête (18, 88), en vue de l'implantation d'un trocart (126).

15. Dispositif selon l'une des revendications 1 à 14, **caractérisé par** la présence d'un mécanisme d'éjection (125), conçu pour éjecter la pièce d'ancrage (110, 112) à présence minimale.

16. Dispositif selon l'une des revendications 1 à 15, équipé d'une pièce d'ancrage conçue pour être utilisée avec ce dispositif, et pourvue d'un corps (32) réalisé en forme de bande.

17. Dispositif équipé d'une pièce d'ancrage, selon la revendication 16, **caractérisé par le fait que** le corps en forme de bande se présente comme un tronçon tubulaire et est doté d'un profil géométrique du tube extérieur (20, 90).

18. Dispositif équipé d'une pièce d'ancrage, selon la revendication 16 ou 17, **caractérisé par le fait que** la pièce d'ancrage (30 ; 110, 112) est de réalisation arrondie aux deux extrémités.

19. Dispositif équipé d'une pièce d'ancrage, selon l'une des revendications 16 à 18, **caractérisé par le fait que** la pièce d'ancrage (30 ; 110, 112) est munie d'au moins un orifice (34, 36) approximativement central, en vue de l'engagement d'un fil de traction (38).

20. Dispositif équipé d'une pièce d'ancrage, selon l'une des revendications 16 à 19, **caractérisé par le fait que** la pièce d'ancrage (30 ; 110, 112) est munie d'au moins un orifice d'extrémité (40, 42), en vue de l'engagement d'un fil de sauvegarde (44).
